# EUROPÄISCHE PATENTSCHRIFT

(11) **EP 2 451 855 B1**
(45) Veröffentlichungstag und Bekanntmachung des Hinweises auf die Patenterteilung: **05.06.2013**
(21) Anmeldenummer: 10729906.7
(22) Anmeldetag: 09.07.2010
(51) Int. Cl.: C08G 18/36, C09D 175/04

(54) **VERFAHREN ZUR HERSTELLUNG VON POLYOLEN AUF BASIS NACHWACHSENDER ROHSTOFFE**
PROCESS FOR PREPARING POLYOLS FROM RENEWABLE RAW MATERIALS
PROCÉDÉ DE PRÉPARATION DE POLYOLS À PARTIR DE MATIÈRES PREMIÈRES RENOUVELABLES

(30) Priorität: 10.07.2009 EP 09165143
(43) Veröffentlichungstag der Anmeldung: 16.05.2012
(73) Patentinhaber: BASF SE, 67056 Ludwigshafen (DE)
(72) Erfinder: KUNST, Andreas, 67063 Ludwigshafen (DE); SCHELPER, Michael, 67065 Ludwigshafen (DE); TELES, Joaquim Henrique, 67166 Otterstadt (DE); ELING, Berend, 49448 Lemförde (DE); REUBER, Jenny, 68167 Mannheim (DE); TEBBEN, Gerd-Dieter, Dr., 68239 Mannheim (DE)
(86) Internationale Anmeldenummer: PCT/EP2010/059850
(87) Internationale Veröffentlichungsnummer: WO 2011/003991

(56) Entgegenhaltungen:
- EP-A2- 0 170 274
- US-A1- 2007 123 725
- SELS, BERT F.: "Solvent- and Metal-Free Ketonization of Fatty Acid Methyl Esters and Triacylglycerols with Nitrous Oxide" ADVANCED SYNTHESIS & CATALYSIS, Bd. 349, Nr. 10, 2. Juli 2007 (2007-07-02) , Seiten 1604-1608, XP002597827 Wiley DOI: DOI: 10.1002/adsc.200600645 in der Anmeldung erwähnt

## Beschreibung

Gegenstand der Erfindung ist ein Verfahren zur Herstellung von Polyolen auf Basis natürlicher Öle, insbesondere für die Herstellung von Polyurethanen.

Polyurethane werden in vielen technischen Gebieten eingesetzt. Ihre Herstellung erfolgt üblicherweise durch Umsetzung von Polyisocyanaten mit Verbindungen mit mindestens zwei mit Isocyanatgruppen reaktiven Wasserstoffatomen in Gegenwart von Treibmitteln sowie gegebenenfalls Katalysatoren und üblichen Hilfs- und/oder Zusatzstoffen.

In neuerer Zeit gewinnen Polyurethan-Ausgangskomponenten auf Basis von nachwachsenden Rohstoffen an Bedeutung. Insbesondere bei den Verbindungen mit mindestens zwei mit Isocyanatgruppen reaktiven Wasserstoffatomen können natürliche Öle und Fette zum Einsatz kommen, die üblicherweise vor dem Einsatz in Polyurethan-Anwendungen chemisch modifiziert werden, um mindestens zwei mit Isocyanatgruppen reaktive Wasserstoffatome einzuführen. Bei den chemischen Modifikationen werden zumeist natürliche Fette und/oder Öle hydroxyfunktionalisiert und gegebenenfalls in einem oder mehreren weiteren Schritten modifiziert. Als Beispiele für Anwendungen von hydroxy-funktionalisierten Fett- und/oder Ölderivaten in PU-Systemen seien beispielsweise WO 2006/116456 und WO2007/130524, genannt.

Die für den Einsatz in der Polyurethanindustrie notwendigen reaktiven Wasserstoffatome müssen wie oben beschrieben mittels chemischer Verfahren in den meisten natürlich vorkommenden Öle eingeführt werden. Hierzu existieren nach dem Stand der Technik im Wesentlichen Verfahren, die sich die in den Fettsäureestern zahlreicher Öle vorkommenden Doppelbindungen zunutze machen. Zum einen können Fette durch Umsetzung mit Percarbonsäuren in Gegenwart eines Katalysators zu den entsprechenden Fett- bzw. Fettsäureepoxiden oxidiert werden. Die anschließende säure- oder basenkatalysierte Ringöffnung der Oxiranringe in Gegenwart von Alkoholen, Wasser, Carbonsäuren, Halogenen oder Halogenwasserstoffen führt zu Bildung von hydroxy-funktionalisierten Fetten bzw. Fettderivaten, beispielsweise beschrieben in WO 2007/127379 und US 2008076901. Der Nachteil dieses Verfahrens besteht darin, dass für den ersten Reaktionsschritt (Epoxidierung) sehr korrosionsbeständige Materialen verwendet werden müssen, da dieser großindustriell mit korrosiver Perameisensäure oder mit Peressigsäure durchgeführt wird. Darüber hinaus muss die anfallende verdünnte Percarbonsäure nach der Herstellung für ein wirtschaftliches Verfahren wieder destillativ aufkonzentriert und zurückgeführt werden, was den Einsatz korrosionsbeständiger und damit energie- und kostenintensiver Destillationsapparaturen erforderlich macht.

Eine weitere Möglichkeit der Hydroxyfunktionalisierung besteht darin, das ungesättigte Fett bzw. Fettsäurederivat im ersten Reaktionsschritt in Gegenwart eines Cobalt- oder Rhodium-haltigen Katalysator mit einem Gemisch aus Kohlenmonoxid und Wasserstoff (Synthesegas) zunächst zu hydroformylieren und anschließend die mit diesem Reaktionsschritt eingeführten Aldehydfunktionen mit einem geeigneten Katalysator (z.B. Raney-Nickel) zu Hydroxygruppen zu hydrieren (vgl. WO 2006/12344 A1 oder auch J. Mol. Cat. A, 2002,184, 65 und J. Polym. Environm. 2002, 10, 49). Bei diesem Reaktionsweg ist jedoch zu beachten, dass zumindest auch für den ersten Reaktionsschritt der Hydroformylierung die Verwendung eines Katalysators und eines Lösungsmittels notwendig ist, die für eine wirtschaftliche Herstellung ebenfalls wieder zurückgewonnen und aufgereinigt bzw. regeneriert werden müssen.

EP1170274A1 beschreibt ein Verfahren zur Herstellung von Hydroxyölen, indem ungesättigte Öle in Gegenwart von Luftsauerstoff oxidiert werden. Nachteilig ist, dass mit diesem Verfahren keine hohen Funktionalisierungsgrade erreicht werden können und dass die Umsetzungen bei hohen Temperaturen erfolgen muss, was zur teilweisen Zersetzung der Fettstruktur führt.

Eine weitere Möglichkeit, Hydroxyfunktionen in Fette einzuführen, besteht darin, dass Fett bzw. das Fettderivat in Gegenwart von Ozon zu spalten, und anschließend zum Hydroxyfettderivat zu reduzieren (vgl. Biomacromolecules 2005, 6, 713; J. Am. Oil Chem. Soc. 2005, 82, 653 und J. Am. Oil Chem. Soc. 2007, 84, 173). Auch dieser Prozess muss in einem Lösungsmittel erfolgen und wird üblicherweise bei niedrigen Temperaturen (-10 bis 0°C) durchgeführt, was ebenfalls in vergleichsweise hohe Fertigungskosten resultiert. Die sicherheitstechnischen Charakteristika dieses Prozesses erfordern darüber hinaus die kostenintensive Bereitstellung von Sicherheitsmaßnahmen, wie Mess- und Regeltechnik oder Kammerung.

In Adv. Synth. Catal. 2007, 349, 1604 wird die Ketonisierung von Fetten mittels Lachgas beschrieben. Die Ketongruppen können unter Verwendung von homogenen Katalysatoren in Hydroxylgruppen umgewandelt werden. Es findet sich jedoch keinerlei Hinweis auf die Weiterverarbeitung dieser Produkte.

Eine Möglichkeit der Herstellung von Polyolen auf Basis nachwachsender Rohstoffe für Polyurethane besteht darin, ungesättigte natürlich vorkommende Fette wie z.B. Sojabohnenöl, Sonnenblumenöl, Rapsöl, etc. oder entsprechende Fettderivate wie Fettsäuren oder deren Monoester durch entsprechende Derivatisierung zu hydroxyfunktionalisierten Fetten bzw. Fettsäurederivaten umzusetzen. Diese Materialien können entweder direkt für die entsprechende PU-Anwendung verwendet werden oder alternativ nach zusätzlicher Anlagerung von Alkylenoxiden an die OH-Funktionen im hydroxyfunktionalisiertem Fett bzw. Fettderivat. Beispiele für die Umsetzung von Hydroxyfettderivaten mit Alkylenoxiden und der Einsatz der Reaktionsprodukte in Polyurethananwendungen sind beispielsweise in WO 2007/143135 und EP1537159 zu finden. Die Anlagerung erfolgt hierbei in den meisten Fällen mit Hilfe sogenannter Doppelmetallcyanid-Katalysatoren.

Die Aufgabe der vorliegenden Erfindung war es, Polyole auf Basis nachwachsender Rohstoffe, insbesondere auf Basis natürliche Fette und Fettsäurederivate, für Polyurethananwendungen bereitzustellen, die kostengünstig erhältlich sind und bei denen durch sehr einfache Anpassung der Reaktionsparameter unterschiedlichste Funktionalitäten abgedeckt werden können und die Produkte somit für einen breiten Anwendungsbereich zur Verfügung stehen. Insbesondere sollte die Herstellung der Öle und Fette nach einem einfachen Verfahren ohne die Verwendung kostspieliger Rohstoffe (Katalysatoren und Lösungsmittel) möglich sein. Dabei sollte die Abtrennung von Katalysatoren vom Reaktionsprodukt auf einfachem Wege möglich sein.

Die Aufgabe konnte gelöst werden, indem ungesättigte natürliche Fette wie Sojabohnenöl, Sonnenblumenöl, Rapsöl, Rizinusöl oder entsprechende Fettsäurederivate in einem ersten Schritt in Gegenwart von Distickstoffmonoxid, auch als Lachgas bezeichnet, zu ketonisierten Fetten bzw. Fettsäurederivaten oxidiert werden, und diese in einem weiteren Reaktionsschritt in Gegenwart von Wasserstoff und einem heterogenen Katalysator zu Hydroxyfetten reduziert werden.

Gegenstand der Erfindung ist demzufolge ein Verfahren zur Herstellung von Polyolen auf Basis nachwachsender Rohstoffe, umfassend die Schritte
a) Umsetzung von ungesättigten natürlichen Fetten, ungesättigten natürlichen Fettsäuren und/oder Fettsäureestern mit Distickstoffmonoxid,
b) Umsetzung des in Schritt a) erhaltenen Produkts mit Wasserstoff unter Verwendung eines heterogenen Katalysators.

Diese Materialien können direkt als Polyolkomponente in verschiedensten Anwendungen, z.B. in der entsprechenden PU-Anwendung eingesetzt werden.

Bevorzugt sind die natürlichen, ungesättigten Fette, ausgewählt aus der Gruppe, enthaltend Rizinusöl, Traubenkernöl, Schwarzkümmelöl, Kürbiskernöl, Borretschsamenöl, Sojaöl, Weizenkeimöl, Rapsöl, Sonnenblumenöl, Erdnussöl, Aprikosenkernöl, Pistazienkernöl, Mandelöl, Olivenöl, Macadamianussöl, Avocadoöl, Sanddornöl, Sesamöl, Hanföl, Haselnussöl, Nachtkerzenöl, Wildrosenöl, Distelöl, Walnussöl, Palmöl, Fischöl, Kokosnussöl, Tallöl, Maiskeimöl, Leinsamenöl.

Bevorzugte sind die Fettsäuren und Fettsäureester ausgewählt aus der Gruppe, enthaltend Myristoleinsäure, Palmitoleinsäure, Ölsäure, Vaccensäure, Petroselinsäure, Gadoleinsäure, Erucasäure, Nervonsäure, Linolsäure, α- und γ-Linolensäure, Stearidonsäure, Arachidonsäure, Timnodonsäure, Clupanodonsäure und Cervonsäure, sowie deren Estern.

Als Fettsäureester können sowohl voll- als auch teilveresterte ein- oder mehrwertige Alkohole verwendet werden. Als ein- oder mehrwertige Alkohole kommen Methanol, Ethanol, Propanol, iso-Propanol, Butanol, Ethylenglykol, Propylenglykol, Diethylenglykol, Dipropylenglykol, Glycerin, Trimethylolpropan, Pentaerythrit, Sorbitol, Saccharose und Mannose in Betracht.

Besonders bevorzugt sind die natürlichen, ungesättigten Fette, ausgewählt aus der Gruppe, enthaltend Rizinusöl, Soja-, Palm-, Sonnenblumen- und Rapsöl. Insbesondere werden Soja-, Palm-, Sonnenblumen- und Rapsöl, eingesetzt. Diese Verbindungen werden im großtechnischen Maßstab insbesondere auch für die Biodieselproduktion verwendet.

Neben den genannten Ölen können auch solche Öle verwendet werden, die aus gentechnisch veränderten Pflanzen erhalten wurden und eine andere Fettsäurezusammensetzung aufweisen. Neben den genannten Ölen können, wie oben beschrieben, ebenfalls die entsprechenden Fettsäuren oder Fettsäureester verwendet werden.

Die Reaktionsschritte a) und b) können unabhängig voneinander und gegebenenfalls auch zeitlich und örtlich getrennt durchgeführt werden. Es ist jedoch möglich, drei Verfahrensschritte unmittelbar nacheinander durchzuführen. Dabei ist es auch möglich, das Verfahren völlig kontinuierlich durchzuführen.

Schritt a) wird vorzugsweise unter Druck, insbesondere in einem Druckbereich von 10 bis 300 bar, und erhöhter Temperatur, insbesondere in einem Temperaturbereich von 200 bis 350 °C, durchgeführt. Dabei kann das Öl bzw. Fett in Substanz oder in Lösungen von geeigneten Lösungsmitteln, wie Cyclohexan, Aceton oder Methanol, eingesetzt werden. Die Umsetzung kann in einem Rührreaktor beliebiger Bauform oder einem Rohrreaktor stattfinden; eine Umsetzung in einem beliebigen anderen Reaktorsystemen ist prinzipiell möglich. Das verwendete Lachgas kann als Reinstoff oder als Mischung mit unter den Reaktionsbedingungen inerten Gasen, wie Stickstoff, Helium, Argon oder Kohlendioxid verwendet werden. Dabei beträgt die Menge der inerten Gase maximal 50 Vol.-%.

Nach Beendigung der Reaktion wird das Reaktionsgemisch für die weitere Verarbeitung abgekühlt, wenn notwendig das Lösungsmittel entfernt, beispielsweise mittels Destillation oder Extraktion, und dem Schritt b) mit oder ohne weitere Aufarbeitung zugeführt.

Das Reaktionsprodukt aus Schritt a) wird in Schritt b) hydriert. Auch dies erfolgt nach üblichen und bekannten Verfahren. Dazu wird die vorzugsweise gereinigte organische Phase aus Schritt a), vorzugsweise in Gegenwart eines geeigneten Lösungsmittels, mit Wasserstoff umgesetzt. Dazu wird die organische Phase, bei einem Druck von 50 bis 300 bar, insbesondere bei 90 bis 150 bar und einer Temperatur von 50 bis 250 °C, insbesondere 50 bis 120 °C in Anwesenheit von Hydrierkatalysatoren umgesetzt. Als Hydrierkatalysatoren werden heterogene Katalysatoren eingesetzt. Vorzugsweise werden Ruthenium enthaltende Katalysatoren verwendet. Die Katalysatoren können außer Ruthenium noch weitere Metalle enthalten, beispielsweise Metalle der Gruppen 6-11 wie z.B. Nickel, Kobalt, Kupfer, Molybdän, Palladium oder Platin.

Vorzugsweise sind die Katalysatoren auf Träger aufgebracht. Als Träger können die üblichen Träger, wie Aluminiumoxid oder Zeolithe eingesetzt werden. In einer bevorzugten Ausführungsform der Erfindung wird als Trägermaterial Kohlenstoff eingesetzt.

Die Katalysatoren können wasserfeucht sein. Die Hydrierung wird vorzugsweise im Festbett durchgeführt.

Nach der Hydrierung werden die organischen Lösungsmittel, der Katalysator und falls erforderlich Wasser abgetrennt. Das Produkt wird, sofern erforderlich, gereinigt.

Die Polyole aus dem Prozessschritt b) haben abhängig von der Art des verwendeten Fetts oder Fettderivates in Prozessschritt a) eine mittlere Funktionalität von 2 bis 6, insbesondere von 2 bis 4 und eine Hydroxylzahl im Bereich zwischen 50 und 300 mg KOH/g. Die Strukturen eignen sich insbesondere für die Herstellung von Polyurethanen, insbesondere für Polyurethan-Weichschaumstoffen, Polyurethan-Hartschaumstoffen und Polyurethan-Beschichtungen. Bei der Herstellung von Polyurethan-Hartschaumstoffen und Polyurethan-Beschichtungen ist es prinzipiell auch möglich, solche Polyole einzusetzen, an die keine Alkylenoxide angelagert wurden, das heißt, Polyole auf Basis von nachwachsenden Rohstoffen, bei deren Herstellung nur die Verfahrensschritte a) und b) durchgeführt wurden. Bei der Herstellung von Polyurethan-Weichschaumstoffen führen derartige Verbindungen auf Grund ihrer geringen Kettenlängen zu einer unerwünschten Vernetzung und sind daher weniger geeignet.

Die Herstellung der Polyurethane erfolgt durch Umsetzung der nach dem erfindungsgemäßen Verfahren hergestellten Polyole mit Polyisocyanaten.

Die Herstellung der erfindungsgemäßen Polyurethane erfolgt durch Umsetzung von Polyisocyanaten mit Verbindungen mit mindestens zwei mit Isocyantgruppen reaktiven Wasserstoffatomen. Bei der Herstellung der Schaumstoffe erfolgt die Umsetzung in Anwesenheit von Treibmitteln.

Zu den eingesetzten Ausgangsverbindungen ist im Einzelnen folgendes zu sagen.

Als Polyisocyanate kommen die an sich bekannten aliphatischen, cycloaliphatischen, araliphatischen und vorzugsweise aromatischen mehrwertigen Isocyanate in Betracht.

Im einzelnen seien beispielhaft genannt: Alkylendiisocyanate mit 4 bis 12 Kohlenstoffatomen im Alkylenrest, wie z.B. Hexamethylen-diisocyanat-1,6; cycloaliphatische Diisocyanate, wie z.B. Cyclohexan-1,3- und -1,4-diisocyanat sowie beliebige Gemische dieser Isomeren, 2,4- und 2,6-Hexahydrotoluylen-diisocyanat sowie die entsprechenden Isomerengemische, 4,4'-, 2,2'- und 2,4'-Dicyclohexylmethan-diisocyanat sowie die entsprechenden Isomerengemische, araliphatische Diisocyanate, wie z.B. 1,4-Xylylen-diisocyanat und Xylylen-diisocyanat-Isomerengemische, vorzugsweise jedoch aromatische Di- und Polyisocyanate, wie z.B. 2,4- und 2,6-Toluylen-diisocyanat (TDI) und die entsprechenden Isomerengemische, 4,4'-, 2,4'- und 2,2'-Diphenylmethan-diisocyanat (MDI) und die entsprechenden Isomerengemische, Mischungen aus 4,4'- und 2,4'-Diphenylmethan-diisocyanaten, Polyphenyl-polymethylen-polyisocyanate, Mischungen aus 4,4'-, 2,4'- und 2,2'-Diphenylmethan-diisocyanaten und Polyphenyl-polymethylenpolyisocyanaten (Roh-MDI) und Mi-schungen aus Roh-MDI und Toluylendiisocyanaten. Die organischen Di- und Polyisocyanate können einzeln oder in Form von Mischungen eingesetzt werden.

Häufig werden auch sogenannte modifizierte mehrwertige Isocyanate, d.h. Produkte, die durch chemische Umsetzung organischer Di- und/oder Polyisocyanate erhalten werden, verwendet. Beispielhaft genannt seien Isocyanurat- und/oder Urethangruppen enthaltende Di- und/oder Polyisocyanate. Im einzelnen kommen beispielsweise in Betracht Urethangruppen enthaltende organische, vorzugsweise aromatische Polyisocyanate mit NCO-Gehalten von 33 bis 15 Gew.-%, vorzugsweise von 31 bis 21 Gew.-%, bezogen auf das Gesamtgewicht des Polyisocyanats.

Die nach dem erfindungsgemäßen Verfahren hergestellten Polyole können in Kombination mit anderen Verbindungen mit mindestens zwei mit Isocyanatgruppen reaktiven Wasserstoffatomen eingesetzt werden.

Als Verbindungen mit mindestens zwei mit Isocyanat reaktiven Wasserstoffatomen, die gemeinsam mit den nach dem erfindungsgemäßen Verfahren hergestellten Polyolen verwendet werden können, kommen insbesondere Polyetheralkohole und/oder Polyesteralkohole zum Einsatz.

Bei der Herstellung von Polyurethan-Hartschaumstoffen wird zumeist mindestens ein Polyetheralkohol eingesetzt, der eine Funktionalität von mindestens 4 und eine Hydroxylzahl größer 250 mgKOH/g aufweist.

Die gemeinsam mit den nach dem erfindungsgemäßen Verfahren hergestellten Polyolen eingesetzten Polyesteralkohole werden zumeist durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, beispielsweise Bernsteinsäure, Glutarsäure, Adipinsäure, Korksäure, Azelainsäure, Sebacinsäure, Decandicarbonsäure, Maleinsäure, Fumarsäure und vorzugsweise Phthalsäure, Isophthalsäure, Terephthalsäure und die isomeren Naphthalindicarbonsäuren, hergestellt.

Die gemeinsam mit den nach dem erfindungsgemäßen Verfahren hergestellten Polyolen verwendeten Polyetheralkohole haben zumeist eine Funktionalität zwischen 2 und 8, insbesondere 4 bis 8.

Insbesondere als Polyhydroxylverbindungen verwendet werden Polyetherpolyole, die nach bekannten Verfahren, beispielsweise durch anionische Polymerisation von Alkylenoxiden in Gegenwart von Alkalimetallhydroxiden, hergestellt werden.

Als Alkylenoxide werden vorzugsweise Ethylenoxid und 1,2-Propylenoxid eingesetzt. Die Alkylenoxide können einzeln, alternierend nacheinander oder als Mischungen verwendet werden.

Als Startermoleküle kommen beispielsweise in Betracht: Wasser, organische Dicarbonsäuren, wie z.B. Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie z.B. gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Anilin, Phenylendiamine, 2,3-, 2,4-, 3,4- und 2,6-Toluylendiamin und 4,4'-, 2,4'- und 2,2'-Diaminodiphenylmethan.

Als Startermoleküle kommen ferner in Betracht: Alkanolamine, wie z.B. Ethanolamin, N-Methyl- und N-Ethylethanolamin, Dialkanolamine, wie z.B. Diethanolamin, N-Methyl- und N-Ethyldiethanolamin und Trialkanolamine wie z.B. Triethanolamin und Ammoniak.

Weiterhin eingesetzt werden mehrwertige, insbesondere zwei- und/oder dreiwertige Alkohole, wie Ethandiol, Propandiol-1,2 und -1,3, Diethylenglykol, Dipropylenglykol, Butandiol-1,4, Hexandiol-1,6, Glycerin, Pentaerythrit, Sorbit und Saccharose, mehrwertige Phenole, wie z.B. 4,4'-Dihydroxydiphenylmethan und 4,4'-Dihydroxydiphenylpropan-2,2, Resole, wie z.B. oligomere Kondensationsprodukte aus Phenol und Formaldehyd und Mannich-Kondensate aus Phenolen, Formaldehyd und Dialkanolaminen sowie Melamin.

Die Polyetherpolyole besitzen eine Funktionalität von vorzugsweise 3 bis 8 und insbesondere 3 und 6 und Hydroxylzahlen von vorzugsweise 120 mgKOH/g bis 770 mgKOH/g und insbesondere 240 mgKOH/g bis 570 mgKOH/g.

Zu den Verbindungen mit mindestens zwei mit Isocyanatgruppen reaktiven Wasserstoffatomen gehören auch die gegebenenfalls mitverwendeten Kettenverlängerer und Vernetzer. Zur Modifizierung der mechanischen Eigenschaften kann sich jedoch der Zusatz von difunktionellen Kettenverlängerungsmitteln, tri- und höherfunktionellen Vernetzungsmitteln oder gegebenenfalls auch Gemischen davon als vorteilhaft erweisen. Als Kettenverlängerungs- und/oder Vernetzungsmittel verwendet werden vorzugsweise Alkanolamine und insbesondere Diole und/oder Triole mit Molekulargewichten kleiner als 400, vorzugsweise 60 bis 300.

Sofern zur Herstellung der Polyurethane Kettenverlängerungsmittel, Vernetzungsmittel oder Mischungen davon Anwendung finden, kommen diese zweckmäßigerweise in einer Menge von 0 bis 20 Gew.-%, vorzugsweise 2 bis 5 Gew.-%, bezogen auf das Gewicht der Verbindungen mit mindestens zwei mit Isocyanatgruppen reaktiven Wasserstoffatomen, zum Einsatz.

Als Treibmittel kann beispielsweise Wasser verwendet werden, das mit Isocyanatgruppen unter Abspaltung von Kohlendioxid reagiert. An Stelle von, vorzugsweise jedoch in Kombination mit Wasser können auch sogenannte physikalische Treibmittel eingesetzt werden. Dabei handelt es sich um gegenüber den Einsatzkomponenten inerte Verbindungen, die zumeist bei Raumtemperatur flüssig sind und unter den Bedingungen der Urethanreaktion verdampfen. Vorzugsweise liegt der Siedepunkt dieser Verbindungen unter 110°C, insbesondere unter 80 °C. Zu den physikalischen Treibmitteln zählen auch inerte Gase, die in den in die Einsatzkomponenten eingebracht bzw. in ihnen gelöst werden, beispielsweise Kohlendioxid, Stickstoff oder Edelgase.

Die bei Raumtemperatur flüssigen Verbindungen werden zumeist ausgewählt aus der Gruppe, enthaltend Alkane und/oder Cycloalkane mit mindestens 4 Kohlenstoffatomen, Dialkylether, Ester, Ketone, Acetale, Fluoralkane mit 1 bis 8 Kohlenstoffatomen, und Tetraalkylsilane mit 1 bis 3 Kohlenstoffatomen in der Alkylkette, insbesondere Tetramethylsilan.

Als Beispiele seien genannt Propan, n-Butan, iso- und Cyclobutan, n-, iso- und Cyclopentan, Cyclohexan, Dimethylether, Methylethylether, Methylbutylether, Ameisensäuremethylester, Aceton, sowie Fluoralkane, die in der Troposphäre abgebaut werden können und deshalb für die Ozonschicht unschädlich sind, wie Trifluormethan, Difluormethan, 1,1,1,3,3-Pentafluorbutan, 1,1,1,3,3-Pentafluorpropan, 1,1,1,2-Tetrafluorethan, Difluorethan und Heptafluorpropan. Die genannten physikalischen Treibmittel können allein oder in beliebigen Kombinationen untereinander eingesetzt werden.

Als Katalysatoren werden insbesondere Verbindungen eingesetzt, welche die Reaktion der Isocyanatgruppen mit den mit Isocyanatgruppen reaktiven Gruppen stark beschleunigen. Insbesondere eingesetzt werden organische Metallverbindungen, vorzugsweise organische Zinnverbindungen, wie Zinn(II)-salze von organischen Säuren.

Weiterhin können als Katalysatoren stark basische Amine eingesetzt werden. Beispiele hierfür sind sekundäre aliphatische Amine, Imidazole, Amidine, Triazine sowie Alkanolamine.

Die Katalysatoren können, je nach Erfordernis, allein oder in beliebigen Mischungen untereinander eingesetzt werden.

Als Hilfsmittel und/oder Zusatzstoffe kommen die für diesen Zweck an sich bekannten Stoffe, beispielsweise oberflächenaktive Substanzen, Schaumstabilisatoren, Zellregler, Füllstoffe, Pigmente, Farbstoffe, Flammschutzmittel, Hydrolyseschutzmittel, Antistatika, fungistatisch und bakteriostatisch wirkende Mittel zum Einsatz.

Nähere Angaben über die zur Durchführung des erfindungsgemäßen Verfahrens eingesetzten Ausgangsstoffe, Treibmittel, Katalysatoren sowie Hilfs- und/oder Zusatzstoffe finden sich beispielsweise im Kunststoffhandbuch, Band 7, "Polyurethane" Carl-Hanser-Verlag München, 1. Auflage, 1966, 2. Auflage, 1983 und 3. Auflage, 1993.

Der Vorteil des erfindungsgemäßen Verfahrens gegenüber der Epoxidierung/Ringöffnung bzw. der Hydroformylierung/Hydrierung besteht darin, dass für den Ketonisierungs-Prozess keine Lösungsmittel und keine Katalysatoren benötigt werden. Damit ist ein vergleichsweise kostengünstiger Zugang hydroxyfunktionalisierter Fette bzw. Fettsäurederivate möglich. Zusätzlich besteht der Vorteil, dass durch einfache Anpassung der Reaktionsbedingungen wie Druck, Temperatur und Verweilzeit sehr einfach und gezielt Funktionalitäten eingestellt werden können und somit Materialien zugänglich werden, die sehr breite Anwendungsmöglichkeiten bieten, die auch über Polyurethananwendungen hinaus gehen.

Gegenüber der Epoxidierung und der Ozonolyse bietet dieses Verfahren den Vorteil, Oligohydroxyfette zu generieren, die bei frei einstellbaren Hydroxylierunggrad keine Doppelbindungen mehr enthalten und somit dem gewöhnlichen Alterungsprozess von Fetten nicht mehr unterliegen (Oxidation der DB, "Ranzigwerden"). Im Falle der Epoxidierung bzw. Ozonolyse gelingt dies nur bei vollständigem Umsatz, dies legt jedoch den Funktionalisierungsgrad fest.

Im Vergleich zur Hydroformylierung ermöglicht die Lachgasoxidation die Herstellung von Material mit komplementärer Reaktivität, da hier ausschließlich sekundäre Hydroxygruppen erzeugt werden, während die Hydroformylierung primäre OH-Gruppen erzeugt.

Die Erfindung soll an den nachfolgenden Beispielen näher erläutert werden.

### Beispiel 1: Oxidation von Sojaöl mit Lachgas

In einen Stahlautoklaven mit 1,2 L Fassungsvolumen wurden 260 g Sojaöl gefüllt, der Autoklav verschlossen und mit Stickstoff inertisiert. 50 bar Lachgas wurden aufgepresst, der Rührer auf 700 U/min eingestellt und eingeschaltet und in Folge die Reaktionsmischung auf 220 °C erwärmt. Nach 22 h Laufzeit wurde auf Raumtemperatur abgekühlt, der Rührer ausgeschaltet und langsam auf Umgebungsdruck entspannt. Nach der Entfernung des Lösungsmittels wurde der gelbliche flüssige Austrag analysiert. Analytische Daten: Bromzahl 36 g Brom/100g, Carbonylzahl 173 mg KOH/g, Esterzahl 196 mg KOH/g, Säurezahl 1,8 mg KOH/g. Elementaranalyse: C = 73,6%, H = 10,8%, O = 15,1%.

### Beispiel 2: Oxidation von Sojaöl mit Lachgas

In einen Stahlautoklaven mit 1,2 L Fassungsvolumen wurden 172 g Sojaöl und 172 g Cyclohexan gefüllt, der Autoklav verschlossen und mit Stickstoff inertisiert. 20 bar Lachgas wurden aufgepresst, der Rührer auf 700 U/min eingestellt und eingeschaltet und in Folge die Reaktionsmischung auf 220 °C erwärmt. Nach 36 h Laufzeit wurde auf Raumtemperatur abgekühlt, der Rührer ausgeschaltet und langsam auf Umgebungsdruck entspannt. Nach der Entfernung des Lösungsmittels wurde der gelbliche flüssige Austrag analysiert.

Analytische Daten: Bromzahl 57 g Brom/100g, Carbonylzahl 64 mg KOH/g, Esterzahl 196 mg KOH/g, Säurezahl 1,8 mg KOH/g. Elementaranalyse: C = 75,6%, H = 11,5%, O = 13,4%.

### Beispiel 3: Oxidation von Sojaöl mit Lachgas im Rohrreaktor

In einem Rohrreaktor (Innenvolumen 210 mL, Verweilzeit ca. 50 min) wurden bei 290 °C und 100 bar 130 g/h einer Mischung aus 50 Gew% Sojaöl und 50 Gew% Cyclohexan mit 45 g/h Lachgas zur Reaktion gebracht. Der Reaktionsaustrag wurde in einen Behälter entspannt, der flüssige Anteil des Reaktionsaustrages abgekühlt und das Cyclohexan destillativ entfernt. Der gelbliche flüssige Austrag wurde analysiert. Analytische Daten: Bromzahl 54 g Brom/100g, Carbonylzahl 81 mg KOH/g, Esterzahl 199 mg KOH/g, Säurezahl 2,6 mg KOH/g. Elementaranalyse: C = 75,0%, H = 11,1%, O = 13,7%.

Als Sojaöl wurde in allen Beispielen ein kommerzielles Produkt der Fa. Aldrich mit einer Bromzahl von 80 g Brom/100g, einer Carbonylzahl von 1 mg KOH/100g, einer Verseifungszahl von 192 mg KOH/g und einer Säurezahl von <0,1 mg KOH/g eingesetzt. Die Elementaranalyse ergab C = 77,6%, H = 11,7%, O = 11,0%.

### Beispiel 4: Hydrierung des oxidierten Sojaöls aus Beispiel 2

In einem 300 mL Stahlautoklaven wird eine Lösung von 20 g oxidiertem Sojaöl aus Beispiel 2 (Carbonylzahl 64 mg KOH/100g, OH-Zahl < 5 mg KOH/1g, Bromzahl 57 g Brom/100g) in 100 mL Tetrahydrofuran zusammen mit 2 g eines wasserfeuchten, 5%igen Ruthenium-Katalysators auf einem Kohlenstoffträger vorgelegt. Es wurde auf 120°C erhitzt und 120 bar Wasserstoff wurden aufgepresst. Bei diesen Parametern wurde 12 h lang gerührt. Anschließend wurde die Reaktionsmischung abgekühlt und entspannt. Der Austrag wurde filtriert und das Lösemittel wird destillativ entfernt. Die Analyse des festen (butterartigen) Rückstands ergab eine OH-Zahl von 64, eine Carbonylzahl <5 und eine Bromzahl von <5.

### Beispiel 5: Hydrierung des oxidierten Sojaöls aus Beispiel 3

In einem 300 mL Stahlautoklaven wurde eine Lösung von 20 g oxidiertem Sojaöl (Carbonylzahl = 81, Bromzahl = 54) in 100 mL Tetrahydrofuran zusammen mit 20 g eines wasserfeuchten, auf Al₂O₃ geträgerten Ruthenium-Katalysator (0,5%ig) vorgelegt. Es wurde auf 120°C erhitzt und 100 bar Wasserstoff wurden aufgepresst. Bei diesen Parametern wurde 12 h lang gerührt. Anschließend wurde die Reaktionsmischung abgekühlt und entspannt. Der Reaktionsaustrag wurde filtriert und danach das Lösemittel destillativ entfernt. Die Analyse des festen (butterartigen) Rückstands ergab eine OH-Zahl von 80, eine Carbonylzahl <5 und eine Bromzahl von <5.

Das Polyol aus Beispiel 5 wurde erfolgreich in einer Polyurethan-Beschichtung-Rezeptur eingesetzt. Dabei wurde festgestellt, dass die Beschichtung sich durch eine sehr hohe Hydrophobie auszeichnet.

### Beispiel 6: Hydrierung des oxidierten Sojaöls aus Beispiel 1

In einem 300 mL Stahlautoklaven wurde eine Lösung von 20 g oxidiertem Sojaöl aus Beispiel 1 (Carbonylzahl = 173, OH-Zahl < 5, Bromzahl = 36) in 100 mL Tetrahydrofuran zusammen mit 2 g eines wasserfeuchten, 5%igen Ruthenium-Katalysators auf einem Kohlenstoffträger vorgelegt. Es wurde auf 120°C erhitzt und 120 bar Wasserstoff wurden aufgepresst. Bei diesen Parametern wurde 12 h lang gerührt. Anschließend wurde die Reaktionsmischung abgekühlt und entspannt. Der Austrag wurde filtriert und danach das Lösemittel destillativ entfernt. Die Analyse des festen (butterartigen) Rückstands ergab eine OH-Zahl von 170, eine Carbonylzahl <5 und eine Bromzahl von <5.

Das Polyol aus Beispiel 6 wurde in einer Polyurethan-Hartschaum-Rezeptur eingesetzt. Dabei wurde festgestellt, dass sich das System durch eine ausgezeichnete Verträglichkeit mit dem als Treibmittel eingesetzten Pentan auszeichnete.

## Patentansprüche

1. Verfahren zur Herstellung von Polyolen, umfassend die Schritte
a) Umsetzung von ungesättigten natürlichen Fetten, ungesättigten natürlichen Fettsäuren und/oder Fettsäureestern mit Distickstoffmonoxid,
b) Umsetzung des in Schritt a) erhaltenen Produkts mit Wasserstoff unter Verwendung eines heterogenen Katalysators.

2. Verfahren nach Anspruch 1, **dadurch gekennzeichnet, dass** die ungesättigten natürlichen Fette sowie Fettderivate ausgewählt sind aus der Gruppe, enthaltend Rizinusöl, Traubenkernöl, Schwarzkümmelöl, Kürbiskernöl, Borretschsamenöl, Sojaöl, Weizenkeimöl, Rapsöl, Sonnenblumenöl, Erdnussöl, Aprikosenkernöl, Pistazienkernöl, Mandelöl, Olivenöl, Macadamianussöl, Avocadoöl, Sanddornöl, Sesamöl, Hanföl, Haselnussöl, Nachtkerzenöl, Wildrosenöl, Distelöl, Walnussöl, Palmöl, Fischöl, Kokosnussöl, Tallöl, Maiskeimöl, Leinsamenöl.

3. Verfahren nach Anspruch 1, **dadurch gekennzeichnet, dass** die Fettsäuren und Fettsäureester ausgewählt sind aus der Gruppe, enthaltend Myristoleinsäure, Palmitoleinsäure, Ölsäure, Vaccensäure, Petroselinsäure, Gadoleinsäure, Erucasäure, Nervonsäure, Linolsäure, α-und γ-Linolensäure, Stearidonsäure, Arachidonsäure, Timnodonsäure, Clupanodonsäure und Cervonsäure, sowie deren Estern.

4. Verfahren nach Anspruch 1, **dadurch gekennzeichnet, dass** die ungesättigten natürlichen Fette ausgewählt sind aus der Gruppe, enthaltend Sojaöl, Palmöl, Sonnenblumenöl, Rapsöl und Rizinusöl.

5. Verfahren nach Anspruch 1, **dadurch gekennzeichnet, dass** in Schritt a) das Distickstoffmonoxid im Gemisch mit inerten Gasen eingesetzt wird.

6. Verfahren nach Anspruch 1, **dadurch gekennzeichnet, dass** Schritt b) in Anwesenheit eines Ruthenium enthaltenden Katalysators durchgeführt wird.

7. Verfahren nach Anspruch 1, **dadurch gekennzeichnet, dass** der Katalysator auf einen Träger aufgebracht ist.

8. Verfahren nach Anspruch 1, **dadurch gekennzeichnet, dass** als Träger Kohlenstoff verwendet wird.

9. Verfahren nach Anspruch 1, **dadurch gekennzeichnet, dass** der Katalysator als Festbett eingesetzt wird.

10. Verwendung von nach einem der Ansprüche 1 bis 9 hergestellten Polyolen zur Herstellung von Polyurethanen.

11. Verfahren zur Herstellung von Polyurethanen durch Umsetzung von Polyisocyanaten mit Verbindungen mit mindestens zwei mit Isocyanatgruppen reaktiven Wasserstoffatomen, **dadurch gekennzeichnet, dass** als Verbindungen mit mindestens zwei mit Isocyanatgruppen reaktiven Wasserstoffatomen nach einem der Ansprüche 1 bis 9 hergestellte Polyole eingesetzt werden.

## Claims

1. A method for producing polyols, comprising the steps of
a) reacting unsaturated natural fats, unsaturated natural fatty acid and/or fatty acid esters with dinitrogen monoxide,
b)reacting the product obtained in step a) with hydrogen using a heterogeneous catalyst.

2. The method according to claim 1, **characterized in that** the unsaturated natural fats and fat derivative are selected from the group containing castor oil, grapeseed oil, black cumin oil, pumpkin seed oil, borage seed oil, soybean oil, wheatgerm oil, rapeseed oil, sunflower oil, peanut oil, apricot kernel oil, pistachio oil, almond oil, olive oil, macadamia nut oil, avocado oil, seabuckthorn oil, sesame oil, hemp oil, hazelnut oil, primula oil, wild rose oil, safflower oil, walnut oil, palm oil, fish oil, coconut oil, tall oil, corngerm oil, linseed oil.

3. The method according to claim 1, **characterized in that** the fatty acids and fatty acid esters are selected from the group containing myristoleic acid, palmitoleic acid, oleic acid, vaccenic acid, petroselinic acid, gadoleic acid, erucic acid, nervonic acid, linoleic acid, α- and γ-linolenic acid, stearidonic acid, arachidonic acid, timnodonic acid, clupanodonic acid, and cervonic acid, and also esters thereof.

4. The method according to claim 1, **characterized in that** the unsaturated natural fats are selected from the group containing soybean oil, palm oil, sunflower oil, rapeseed oil and castor oil.

5. The method according to claim 1, **characterized in that** in step a) the dinitrogen monoxide is used in a mixture with insert gases.

6. The method according to claim 1, **characterised in that** step b) is carried out in the presence of a catalyst comprising ruthenium.

7. The method according to claim 1, **characterised in that** the catalyst is applied on a support.

8. The method according to claim 1, **characterised in that** carbon is used as support.

9. The method according to claim 1, **characterized in that** the catalyst is used as a fixed bed.

10. The use of polyols produced according to any of claim 1 to 9 for preparing polyurethanes.

11. A method for preparing polyurethanes by reacting polyisocyanates with compounds having at least two hydrogen atoms that are reactive with isocyanate groups, **characterized in that** polyols produced according to any of claims 1 to 9 are used as
compounds heaving at least two hydrogen atoms that are reactive with isocyanate groups.

## Revendications

1. Procédé de préparation de polyols, comprenant les étapes suivantes :
a) la mise en réaction de graisses naturelles insaturées, d'acides gras naturels insaturées et/ou d'esters d'acides gras avec du monoxyde de diazote,
b) la mise en réaction du produit obtenu à l'étape a) avec de l'hydrogène en utilisant un catalyseur hétérogène.

2. Procédé selon la revendication 1, **caractérisé en ce que** les graisses naturelles insaturées et les dérivés de graisses sont choisis dans le groupe contenant l'huile de ricin, l'huile de pépin de raisin, l'huile de nigelle, l'huile de pépin de courge, l'huile de bourrache, l'huile de soja, l'huile de germe de blé, l'huile de colza, l'huile de tournesol, l'huile d'arachide, l'huile de noyau d'abricot, l'huile de pistache, l'huile d'amande, l'huile d'olive, l'huile de macadamia, l'huile d'avocat, l'huile d'argousier, l'huile de sésame, l'huile de chanvre, l'huile de noisette, l'huile d'onagre, l'huile de rose sauvage, l'huile de carthame, l'huile de noix, l'huile de palme, l'huile de poisson, l'huile de noix de coco, le tallöl, l'huile de maïs, l'huile de lin.

3. Procédé selon la revendication 1, **caractérisé en ce que** les acides gras et les esters d'acides gras sont choisis dans le groupe contenant l'acide myristoléique, l'acide palmitoléique, l'acide oléique, l'acide vaccénique, l'acide pétrosélinique, l'acide gadoléique, l'acide érucique, l'acide nervonique, l'acide linoléique, l'acide α- et γ-linolénique, l'acide stéaridonique, l'acide arachidonique, l'acide timnodonique, l'acide clupanodonique et l'acide cervonique, ainsi que leurs esters.

4. Procédé selon la revendication 1, **caractérisé en ce que** les graisses naturelles insaturées sont choisies dans le groupe contenant l'huile de soja, l'huile de palme, l'huile de tournesol, l'huile de colza et l'huile de ricin.

5. Procédé selon la revendication 1, **caractérisé en ce qu'**à l'étape a), le monoxyde de diazote est utilisé en mélange avec des gaz inertes.

6. Procédé selon la revendication 1, **caractérisé en ce que** l'étape b) est réalisée en présente d'un catalyseur contentant du ruthénium.

7. Procédé selon la revendication 1, **caractérisé en ce que** le catalyseur est appliqué sur un support.

8. Procédé selon la revendication 1, **caractérisé en ce que** du carbone est utilisé en tant que support.

9. Procédé selon la revendication 1, **caractérisé en ce que** le catalyseur est utilisé sous la forme d'un lit fixe.

10. Utilisation de polyols préparés selon l'une quelconque des revendications 1 à 9 pour la réparation de polyuréthannes.

11. Procédé de préparation de polyuréthanes par misse en réaction de polyisocyanates avec des composés contenant au moins deux atomes d'hydrogène réactifs avec les groupes isocyanate, **caractérisé en ce que** des polyols préparés selon l'une quelconque des revendications 1 à 9 sont utilisés en tant que composés contenant au moins deux atomes d'hydrogène réactifs avec les groupes isocyanate.
